# EUROPÄISCHE PATENTSCHRIFT

(11) **EP 0 275 421 B1**
(45) Veröffentlichungstag und Bekanntmachung des Hinweises auf die Patenterteilung: **28.09.1994**
(21) Anmeldenummer: 87117676.4
(22) Anmeldetag: 30.11.1987
(51) Int. Cl.: C07D 233/14

(54) **Verfahren zur Herstellung dünnflüssiger Amphotenside**
Process for the preparation of highly viscous amphotensides
Procédé de préparation d'amphotenside fluide

(30) Priorität: 08.12.1986 DE 3641871
(43) Veröffentlichungstag der Anmeldung: 27.07.1988
(73) Patentinhaber: Henkel Kommanditgesellschaft auf Aktien, 40191 Düsseldorf (DE)
(72) Erfinder: Ploog, Uwe, Dr., D-5657 Haan (DE); Uphues, Günter, D-4019 Monheim (DE); Nikolaus, Peter, Dr., D-4010 Hilden (DE)

(56) Entgegenhaltungen:
- EP-A- 0 001 006
- EP-A- 0 040 346
- DE-A- 2 063 424
- US-A- 2 528 378
- US-A- 2 773 068
- US-A- 4 212 983

## Beschreibung

Die vorliegende Erfindung betrifft ein Verfahren zur Herstellung dünnflüssiger Amphotenside durch Quaternierung diamidarmer Imidazoline, die durch Umsetzung von Fettsäuren mit Aminoethylethanolamin erhältlich sind.

US-PS 4 212 983 beschreibt die Herstellung eines diamidarmen Imidazolins durch Erhitzen des rohen Imidazolins mit einem Überschuß an Aminoethylethanolamin durch die Behandlung des Reaktionsgemisches im Vakuum unter Rückflußbedingungen.

Die Herstellung von Imidazolin-Acrylat-Addukten wird in US-PS 3 555 041 beschrieben. Die entsprechenden Basisimidazoline werden durch azeotrope Umsetzung von Fettsäure und Amin hergestellt.

US-PS 4 058 488 betrifft die Herstellung von Imidazolinoxiden, wobei die Basisimidazoline unter anderem durch azeotrope Umsetzung hergestellt werden.

Durch Umsetzung mit einem Quaternierungsmittel können aus den Imidazolinen die entsprechenden amphoteren Tenside erhalten werden.

US-PS 2 528 378 beschreibt die Umsetzung von äquimolaren Mengen Imidazolin und Chloressigsäure in einer wässrigen Lösung von überschüssigem Alkali. Es wird eine feste Masse erhalten.

Ebenso feste Produkte werden nach der US-PS 2 773 068 erhalten, nach der man eine alkalische Natriumchloracetatlösung vorlegt und anschließend in Gegenwart von einem Überschuß an Natronlauge Imidazolin mit dem doppelten Überschuß an Chloressigsäure umsetzt.

GB-PS 850 514 beschreibt die hydrolytische Spaltung von Imidazolin zum linearen Aminoamid, das mit Natriumchloracetat umgesetzt und mit Ethersulfat ausgemischt wird. Die GB-PS beschreibt somit die Umsetzung von hydrolysiertem Imidazolin mit Natriumchloracetat.

Einen hohen Anteil an freier Chloressigsäure enthaltende Produkte beschreibt die DE-OS 20 63 424 (GB-PS 1 352 770). Imidazolin und Chloressigsäure werden vorgelegt und bei erhöhter Temperatur bei annähernd neutralem pH-Wert mit Natronlauge versetzt.

Die GB-PS 930 296 beschreibt die Umsetzung von äquimolaren Mengen Imidazolin und Chloressigsäure, die mit einem leichten Überschuß an Natronlauge neutralisiert wird. Diese Umsetzung wird bei 0 bis 15°C durchgeführt, anschließend auf 95°C erhitzt und dann eine weitere Teilmenge an Natronlauge zugegeben. Die Umsetzung erfolgt in Gegenwart überschüssiger Natronlauge; formuliert werden Carboxymethylierungsprodukte auf der Basis eines linearen und verzweigten Aminoamides. Die sehr niedrige Temperatur bei Beginn der Reaktion soll den Einfluß des Überschusses an Natronlauge auf die Ringspaltung vermindern.

EP-PS 1 006 beschreibt die Umsetzung eines Imidazolins bei 40 bis 90°C und pH 7 bis 11,5. Wenn 0,5 Mol Carboxymethylierungssubstanz reagiert haben, wird der pH-Wert auf 9 bis 12 angehoben. Die Reaktionskontrolle wird gemäß der allgemeinen Formel 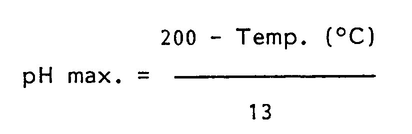
durchgeführt. Das aufwendige Verfahren liefert Produkte mit Gehalten von mehr als 1 % an freier Chloressigsäure.

US-PS 4 269 730 beschreibt in Spalte 1 die Umsetzung von Imidazolin mit neutralisierter Chloressigsäure in Abwesenheit von starkem Alkali. In Spalte 2 wird die Forderung nach genauer Einhaltung der Reihenfolge, d.h. erst Umsetzung ohne Alkali, dann Alkalizugabe, aufgestellt.

Beansprucht wird die Erhitzung von 1 Mol Imidazolin mit mindestens 1 Mol Natriumchloracetat in Wasser (mit 20 bis 50 % Feststoffgehalt) bei 70 bis 80°C, bis nahezu das gesamte Natriumchloracetat verbraucht ist und die Zugabe einer äquivalenten Menge Natronlauge und anschließende Erhitzung auf 70 bis 80°C, bis die Ringöffnung eingetreten ist. Imidazolin und Chloracetat werden in einem aufwendigen, genau dosierten Verfahren derart miteinander zur Reaktion gebracht, daß sich Natriumchloracetat immer im Überschuß befindet.

In Spalte 13 wird ein 92 bis 99,5 %iges Imidazolin gefordert, das weitgehend frei ist von Ausgangsmethylester (0 bis 2 %) und lediglich bis zu 5 % Aminoamid ("monoamide") enthalten darf; Diamidgehalte werden nicht erwähnt.

In der EP-PS 40 346 wird ein Verfahren zur Beseitigung des bei der Imidazolinbildung gleichzeitig entstehenden Diamids beschrieben, das in dem fertigen Amphotensid besonders hartnäckige Trübungen erzeugt. Zur Zerstörung des Diamids wird das Imidazolin vor der Umsetzung mit Natriumchloracetat alkalisch behandelt und damit gezielt in lineares Aminoamid überführt. Das resultierende Amphotensid ist trübungsfrei, jedoch hochviskos.

Die Aufgabe der vorliegenden Erfindung bestand darin, ein neues Verfahren zur Herstellung von hochkonzentrierten dünnflüssigen Amphotensiden bereitzustellen, das die geschilderten Nachteile nicht aufweist. Die Aufgabe konnte durch ein Quarternierungsverfahren gelöst werden, das von diamidarmen Imidazolinen ausgeht und das unter genauer Einhaltung bestimmter Zugabe- und Temperaturparameter durchgeführt wird.

Gegenstand der Erfindung ist ein Verfahren zur Herstellung dünnflüssiger Amphotenside durch Quaternierung diamidarmer Imidazoline, das dadurch gekennzeichnet ist, daß man
(a) pro 1 Mol Imidazolin, das wenigstens 80 % Imidazolin und höchstens 3 % Diamidgehalt aufweist, 1 bis 3 Mol eines neutralisierten Quaternierungsmittels gelöst in Wasser vorlegt,
(b) das geschmolzene Imidazolin innerhalb von wenigstens 1 h mit steigender Geschwindigkeit zu der auf 55 bis 65 °C erwärmten Quaternierungsmittellösung unter Beibehaltung des Temperaturbereichs zugibt,
(c) nach Beendigung der Zugabe des Imidazolins die Temperatur weitere 80 bis 100 min im Bereich von 55 bis 65 °C hält,
(d) anschließend für weitere 80 bis 100 min die Temperatur auf einen Bereich von 75 bis 85 °C mit der Maßgabe erhöht, daß das Imidazolin in einem molaren Verhältnis von annähernd 1 : 1 mit dem Quaternierungsmittel reagiert hat,
(e) ein Moläquivalent eines Alkalimetallhydroxides, bezogen auf die vorgelegte Menge an Quaternierungsmittel, innerhalb von höchstens 15 min bei einer Temperatur von wenigstens 80 °C zugibt, wobei ein pH-Wert im Bereich von 11,5 bis 12,0 eingestellt wird und
(f) nach Beendigung der Zugabe die Reaktionsmischung bei einer Temperatur im Bereich von 80 bis 90 °C während einer Zeit von 140 bis 180 min hält.

Bevorzugt werden 1,5 bis 2,5 Mol neutralisiertes Quaternierungsmittel, gelöst in Wasser, vorgelegt.

Als Quaternierungsmittel können Halogencarbonsäuren mit 2 oder 3 Kohlenstoffatomen und ihre Alkalisalze verwendet werden. Natriumchloracetat stellt ein bevorzugtes Quaternierungsmittel dar.

Als bevorzugte Alkalimetallhydroxide werden Kaliumhydroxid und Natriumhydroxid verwendet. Insbesondere verwendet wird Natriumhydroxid in Form einer 50 %igen wässrigen Lösung.

Das Quaternierungsmittel zur Ringöffnung der diamidarmen Imidazoline wird gemäß der vorliegenden Erfindung in vollneutralisierter Form vorgelegt. Der pH-Wert wird in der ersten Stufe nicht durch NaOH-Zugabe kontrolliert, sondern das Imidazolin portionsweise zugegeben und nach der Imidazolinzugabe der pH-Wert auf Werte unter 7 abfallen gelassen; während der NaOH-Zugabe läßt man die Temperatur auf nahezu 100°C ansteigen.

Die für die Durchführung des vorstehend beschriebenen erfindungsgemäßen Verfahrens als Ausgangsmaterial benötigten technischen Imidazoline mit einem Imidazolingehalt von wenigstens 80 % und einem Diamidgehalt von höchstens 3 Gew.-% können mit Hilfe eines neuen Verfahrens durch Kondensation von Fettsäuren mit Aminoethylethanolamin unter speziellen Bedingungen bereitgestellt werden. Dieses neue Verfahren ist dadurch gekennzeichnet, daß man
(a) 1 Mol einer Fettsäure mit 6 bis 22 C-Atomen mit einer äuqimolaren Menge oder einem bis zu 0,3-molaren Überschuß an Aminoethylethanolamin in Gegenwart von 0,05 bis 0,1 % hypophosphoriger Säure, bezogen auf den Gesamtansatz, in einem inerten, mit Wasser azeotrope Gemische bildenden Lösungsmittel unter Rühren bis zur Wasserabspaltung erhitzt,
(b) das gebildete Wasser durch azeotrope Destillation aus dem Reaktionsgemisch abtrennt und
(c) nach Beendigung der Wasserabspaltung das Lösungsmittel mit dem restlichen Amin unter vermindertem Druck abdestilliert.

Als Fettsäuren können beispielsweise Laurinsäure, Capronsäure, Carpylsäure, Caprinsäure, Myristinsäure` Palmitinsäure, Stearinsäure, Arachinsäure, Behensäure, Isostearinsäure, 12-Hydroxystearinsäure, oder deren beliebige Mischungen verwendet werden.

Das bevorzugte Lösungsmittel ist Xylol, jedoch können auch andere Lösungsmittel, die mit Wasser ein Azeotrop bilden, verwendet werden.

Die Vorteile dieses Verfahrens bestehen darin, daß durch die Vornahme des Imidazolinringschlusses unter den Bedingungen einer azeotropen Destillation überraschenderweise ein diamidarmes Imidazolin erhalten wird, dessen Diamidgehalt weniger als 1 % beträgt. Bei üblichen Verfahren ohne Lösungsmittel werden auch bei 50 % Aminüberschuß Diamidwerte von ca. 8 % erhalten.

Die nachfolgend genannten Beispiele beschreiben bevorzugte Ausführungsformen zur Bereitstellung diamidarmer Imidazoline als Zwischenprodukte und deren Umsetzung mit einem Quaternierungsmittel zu amphoteren Tensiden.

### Beispiel 1

### (Herstellung eines diamidarmen Imidazolins)

2 000 g (10 Mol) Laurinsäure, 99 %, und 1 300 g (12,5 Mol) Aminoethylethanolamin wurden in Gegenwart von 3,3 g hypophosphoriger Säure (50 %) und unter Verwendung von ca. 300 ml Xylol in einer Stickstoffatmosphäre unter Rühren bis zur Wasserabspaltung erhitzt. Unter langsamer Temperatursteigerung (geregelt über den Xylolgehalt) von 159 auf 209°C destillierten innerhalb von 4 h 442,7 g eines wässrigen Destillats über, die ca. 104 g (ca. 1 Mol) Amin enthielten. Nach beendeter Wasserabspaltung wurde das Lösungsmittel mit dem restlichen Amin unter Vakuum von 25 mbar abgezogen.

### Kennzahlen des Produktes:

| | |
|---|---|
| N_{Kj} | =10,3 % |
| Nₜᵢₜᵣ | =5,22 % |
| Imidazolingehalt | ca. 99 % (UV-spektroskopisch ermittelt) |
| Diamidgehalt | = 1,07 % (Ionenaustauscherdurchlauf) |

### Beispiel 2

Es wurden 108 g Natriumchloracetat in 220 g Wasser gegeben. Im Verlauf von 20 min wurde die Temperatur der Lösung langsam auf 60°C erhöht und 98 g des gemäß Beispiel 1 hergestellten Imidazolins so zugegeben, daß nach 30 min 25 % und nach 65 min die Gesamtmenge zugegeben waren. Danach wurde 90 min bei der gleichen Temperatur und 120 min bei 80 °C weitergerührt. Anschließend gab man 1 Mol-Äquivalent, bezogen auf die Menge an Natriumchloracetat, an Natronlauge in Form einer 50 %igen wässrigen Lösung im Verlauf von 10 min zu, wodurch ein pH-Wert von 11 erreicht wurde. Im Verlauf von weiteren 120 min wurde die Temperatur der Reaktionsmischung im Bereich von 80 bis 90°C gehalten und anschließend abgekühlt.

Es wurde eine niedrigviskose Lösung mit ca. 45 % Feststoffgehalt erhalten.

### Kennzahlen des Produktes:

| | |
|---|---|
| N_{Kj} | = 2 % |
| Chloressigsäure | = 0,05 % |
| Imidazolingehalt | = 0 % |

### Beispiel 3

Zu einer Lösung aus 389 g Wasser und 234 g Natriumchloracetat, die zuvor auf 60 °C erwärmt worden war, wurden 224 g eines gemäß Beispiel 1 auf der Basis von Kokosfettsäure hergestellten Imidazolins so zugegeben, daß nach 30 min 20 %, nach 60 min 70 % und nach 70 min die Gesamtmenge zugesetzt waren. Danach wurde weitere 90 min bei 60 °C und 90 min bei 80 °C gerührt. Nach der Zugabe von 160 g Natronlauge, 50 %ig innerhalb von 15 min, bei der die Temperatur bis auf 99 °C anstieg, wurde die Reaktion nach weiteren 120 min bei 80 bis
85 °C beendet.
Es wurde eine mittelviskose Flüssigkeit mit 50 % Feststoff erhalten.

### Kennzahlen des Produktes:

| | |
|---|---|
| N_{Kj} | = 2,2 % |
| Chloressigsäure | = 0,1 % |
| Imidazolingehalt | = 0 % |

### Vergleichsbeispiel 1 (analog EP-PS 1 006)

86 g Chloressigsäure wurden in 180 g Wasser gelöst. Bei einer Temperatur von 35 °C gab man 98 g eines gemäß Beispiel 1 hergestellten Imidazolins zu. Dabei erhöhte sich die Temperatur auf 45 °C und es stellte sich ein pH-Wert von 2,67 ein, der im Verlaufe von 65 min durch Zugabe von insgesamt 72,6 g Natronlauge, 50 %ig auf einen Wert von 11,2 angehoben wurde. Gleichzeitig wurde die Temperatur auf 55 °C erhöht. Während weiterer 145 min bei 55 °C wurde der pH-Wert durch laufende Natronlauge-Zugabe bei 10,5 bis 11,5 gehalten. Dazu waren 33,4 g der 50 %igen Lauge erforderlich. Danach wurde die Temperatur auf 70 °C erhöht und während weiterer 200 min ein pH-Wert von 11,5 bis 12,4 durch Zugabe von insgesamt 30,6 g Natronlauge, 50 %ig aufrecht erhalten. Anschließend wurde noch
60 min bei 80 °C gerührt, wobei der pH-Wert auf 10,5 absank.

Es wurde eine viskose Flüssigkeit mit 45 % Feststoff erhalten.

### Kennzahlen des Produktes:

| | |
|---|---|
| N_{Kj} | = 1,97 % |
| Chloressigsäure | = 1,0 % |

### Vergleichsbeispiel 2 (analog DE-OS 20 63 424)

Es wurden 86 g Chloressigsäure in 170 g Wasser vorgelegt und unter Temperaturerhöhung von 14 auf 38°C im Verlauf von 23 min 98 g eines gemäß Beispiel 1 hergestellten Imidazolins zugegeben. Der pH-Wert wurde durch Zugabe von 50 %iger wässriger NaOH im Bereich von 7,0 bis 7,5 eingestellt, wobei die Temperatur von 55°C im Verlauf von 315 min konstant blieb. Nach Zugabe der Restmenge der insgesamt 109 g NaOH im Verlauf von 10 min stieg die Temperatur auf 65°C und der pH-Wert auf 12,7. Im Verlauf von weiteren 15 min sank der pH-Wert der Lösung schnell auf 7,5, wobei die Probe vorübergehend gelartig, bald flüssig und klar wurde. Man beließ weitere 100 min bei 80°C und gab 9,4 g Hexylenglykol zu. Nach dem Abkühlen konnten 92 % des Endproduktes (Verlust durch Probennahmen) erhalten werden.

### Kennzahlen des Produktes:

| | |
|---|---|
| N_{Kj} | = 2,13 % |
| Chloressigsäure | = 3,5 %. |

## Patentansprüche

1. Verfahren zur Herstellung dünnflüssiger Amphotenside durch Quaternierung diamidarmer Imidazoline, dadurch gekennzeichnet, daß man
(a) pro 1 Mol Imidazolin, das wenigstens 80 % Imidazolin und höchstens 3 % Diamidgehalt aufweist, 1 bis 3 Mol eines neutralisierten Quaternierungsmittels gelöst in Wasser vorlegt,
(b) das geschmolzene Imidazolin innerhalb von wenigstens 1 h mit steigender Geschwindigkeit zu der auf 55 bis 65 °C erwärmten Quaternierungsmittellösung unter Beibehaltung des Temperaturbereichs zugibt,
(c) nach Beendigung der Zugabe des Imidazolins die Temperatur weitere 80 bis 100 min im Bereich von 55 bis 65 °C hält,
(d) anschließend für weitere 80 bis 100 min die Temperatur auf einen Bereich von 75 bis 85 °C mit der Maßgabe erhöht, daß das Imidazolin in einem molaren Verhältnis von annähernd 1 : 1 mit dem Quaternierungsmittel reagiert hat,
(e) ein Moläquivalent eines Alkalimetallhydroxides, bezogen auf die vorgelegte Menge an Quaternierungsmittel, innerhalb von höchstens 15 min bei einer Temperatur von wenigstens 80 °C zugibt, wobei ein pH-Wert im Bereich von 11,5 bis 12,0 eingestellt wird und
(f) nach Beendigung der Zugabe die Reaktionsmischung bei einer Temperatur im Bereich von 80 bis 90 °C während einer Zeit von 140 bis 180 min hält.

2. Verfahren nach Anpruch 1, dadurch gekennzeichnet, daß man vorzugsweise 1,5 bis 2,5 mol eines neutralisierten Quaternierungsmittels gelöst in Wasser vorlegt.

3. Verfahren nach Ansprüchen 1 und 2, dadurch gekennzeichnet, daß das Quaternierungsmittel ausgewählt ist aus der aus Halogencarbonsäuren mit 2 oder 3 C-Atomen und ihren Alkalisalzen bestehenden Gruppe.

4. Verfahren nach Anspruch 3, dadurch gekennzeichnet, daß das Quaternierungsmittel Natriumchloracetat ist.

5. Verfahren nach Anspruch 4, dadurch gekennzeichnet, daß das Alkalimetallhydroxid ausgewählt ist aus Kaliumhydroxid und/oder Natriumhydroxid.

6. Verfahren nach Anspruch 5, dadurch gekennzeichnet, daß als Alkalimetallhydroxid Natriumhydroxid in Form einer 50 %igen wäßrigen Lösung verwendet wird.

7. Verfahren nach den Ansprüchen 1 bis 6, dadurch gekennzeichnet, daß man als Ausgangsmaterial diamidarme Imidazoline einsetzt, die dadurch erhältlich sind, daß man
(a) 1 Mol einer Fettsäure mit 6 bis 22 C-Atomen mit einer äquimolaren Menge oder einem bis zu 0,3-molaren Überschuß an Aminoethylethanolamin in Gegenwart von 0,05 bis 0,1 % hypophosphoriger Säure, bezogen auf den Gesamtansatz, in einem inerten, mit Wasser azeotrope Gemische bildenden Lösungsmittel unter Rühren zur Wasserabspaltung erhitzt,
(b) das gebildete Wasser durch azeotrope Destillation aus dem Reaktionsgemisch abtrennt und
(c) nach Beendigung der Wasserabspaltung das Lösungsmittel mit dem restlichen Amin unter vermindertem Druck abdestilliert.

8. Verfahren nach Anspruch 7, dadurch gekennzeichnet, daß die Fettsäure ausgewählt ist aus der aus Laurinsäure, Capronsäure, Caprylsäure, Caprinsäure, Myristinsäure, Palmitinsäure, Stearinsäure, Arachinsäure, Behensäure, Ölsäure, Erucasäure, Isononansäure, Isotridecansäure, Isostearinsäure, 12-Hydroxystearinsäure oder deren beliebigen Mischungen bestehenden Gruppe.

9. Verfahren nach Ansprüchen 7 und 8, dadurch gekennzeichnet, daß das inerte Lösungsmittel aromatisch und/ oder aliphatisch ist und vorzugsweise Xylol ist.

## Claims

1. A process for the production of low-viscosity amphoteric surfactants by quaternization of low-diamide imidazolines, characterized in that
(a) from 1 to 3 mols of a neutralized quaternizing agent dissolved in water are initially introduced per mol of imidazoline containing at least 80% imidazoline and at most 3% diamide,
(b) the molten imidazoline is added at an increasing rate to the quaternizing agent solution heated to 55 - 65°C over a period of at least one hour during which the temperature range is maintained,
(c) after the imidazoline has been added, the temperature is kept at 55 to 65°C for another 80 to 100 minutes,
(d) the temperature is then increased to 75 - 85°C for another 80 to 100 minutes, with the proviso that the imidazoline has reacted with the quaternizing agent in a molar ratio of approximately 1:1,
(e) 1 mol equivalent of an alkali metal hydroxide, based on the quantity of quaternizing agent initially introduced, is added over a period of at most 15 minutes at a temperature of at least 80°C, a pH value of from 11.5 to 12.0 being adjusted and
(f) on completion of the addition, the reaction mixture is kept at a temperature in the range from 80 to 90°C for a period of 140 to 180 minutes.

2. A process as claimed in claim 1, characterized in that from 1.5 to 2.5 mols of a neutralized quaternizing agent dissolved in water are preferably initially introduced.

3. A process as claimed in claims 1 and 2, characterized in that the quaternizing agent is selected from the group consisting of halocarboxylic acids containing 2 or 3 carbon atoms and alkali metal salts thereof.

4. A process as claimed in claim 3, characterized in that the quaternizing agent is sodium chloroacetate.

5. A process as claimed in claim 4, characterized in that the alkali metal hydroxide is selected from potassium hydroxide and/or sodium hydroxide.

6. A process as claimed in claim 5, characterized in that sodium hydroxide in the form of a 50% aqueous solution is used as the alkali metal hydroxide.

7. A process as claimed in claims 1 to 6, characterized in that low-diamide imidazolines obtainable by
(a) heating 1 mol of a C₆₋₂₂ fatty acid with stirring with an equimolar quantity or an up to 0.3 molar excess of aminoethyl ethanolamine in the presence of 0.05 to 0.1% of hypophosphorous acid, based on the mixture as a whole, in an inert solvent forming azeotropic mixtures with water until water is eliminated,
(b) separating the water formed from the reaction mixture by azeotropic distillation and
(c) distilling off the solvent with the residual amine under reduced pressure after the elimination of water has stopped,
are used as starting material.

8. A process as claimed in claim 7, characterized in that the fatty acid is selected from the group consisting of lauric acid, caproic acid, caprylic acid, capric acid, myristic acid, palmitic acid, stearic acid, arachic acid, behenic acid, oleic acid, erucic acid, isononanoic acid, isotridecanoic acid, isostearic acid, 12-hydroxystearic acid or mixtures thereof.

9. A process as claimed in claims 7 and 8, characterized in that the inert solvent is aromatic and/or aliphatic and is preferably xylene.

## Revendications

1. Procédé de préparation d'amphotensides (ou agents de surface ampholytiques) fluides, par quaternisation d'imidazolines pauvres en diamide, caractérisé en ce que l'on
(a) présente par mole d'imidazoline, qui contient au moins 80 % d'imidazoline et une concentration maximale de diamide de 3 %, 1 à 3 moles d'un agent de quaternisation neutralisé dissous dans de l'eau,
(b) ajoute l'imidazoline fondue à la solution d'agent de quaternisation chauffée à 55-65 °C, en l'espace d'au moins 1 h à une vitesse croissante et en respectant la plage de 55 à 65 °C,
(c) maintient la température dans la plage de 55 à 65 °C pendant encore 80 à 100 min après la fin de l'adjonction de l'imidazoline,
d) augmente ensuite la température dans une plage de 75 à 85 °C pendant encore 80 à 100 min, dans la mesure où l'imidazoline a réagi avec l'agent de quaternisation dans un rapport molaire d'à peu près 1:1,
(e) ajoute un équivalent molaire - par rapport à la quantité présentée d'agent de quaternisation - d'un hydroxyde de métal alcalin en l'espace de 15 min au maximum, à une température d'au moins 80 °C, un pH compris dans l'intervalle de 11,5 à 12,0 étant ajusté et
(f) maintient le mélange réactionnel après la fin de l'adjonction à une température comprise dans l'intervalle de 80 à 90 °C pendant une période de 140 à 180 min.

2. Procédé selon la revendication 1, caractérisé en ce que l'on présente de préférence 1,5 à 2,5 moles d'un agent de quaternisation neutralisé dissous dans de l'eau.

3. Procédé selon les revendications 1 et 2, caractérisé en ce que l'agent de quaternisation est sélectionné parmi le groupe constitué des hydracides halogénés comportant 2 ou 3 atomes de C et de leurs sels de métaux alcalins.

4. Procédé selon la revendication 3, caractérisé en ce que l'agent de quaternisation est du chloracétate de sodium.

5. Procédé selon la revendication 4, caractérisé en ce que l'hydroxyde de métal alcalin est sélectionné entre l'hydroxyde de potassium et/ou l'hydroxyde de sodium.

6. Procédé selon la revendication 5, caractérisé en ce que l'on utilise comme hydroxyde de métal alcalin, de l'hydroxyde de sodium sous la forme d'une solution aqueuse à 50 %.

7. Procédé selon les revendications 1 à 6, caractérisé en ce que l'on utilise comme matière de départ des imidazolines pauvres en diamide, qui sont obtenables en
(a) chauffant 1 mole d'un acide gras comportant 6 à 22 atomes de C avec une quantité équimolaire ou avec un excès jusqu'à 0,3-molaire d'aminoéthyléthanolamine en présence de 0,05 à 0,1 % d'acide hypophosphoreux par rapport à la préparation totale, dans un solvant inerte, formant avec l'eau des mélanges azéotropes, sous agitation pour éliminer l'eau,
(b) séparant l'eau formée du mélange réactionnel par distillation azéotrope et
(c) chassant le solvant avec l'amine résiduelle par distillation sous pression réduite après la fin de l'élimination de l'eau.

8. Procédé selon la revendication 7, caractérisé en ce que l'acide gras est sélectionné parmi le groupe constitué des acides laurique, caproïque, caprylique, caprique, myristique, palmitique, stéarique, arachique, béhénique, oléique, érucique, isononanoïque, isotridécanoïque, isostéarique, 12-hydroxystéarique ou de mélanges quelconques de ceux-ci.

9. Procédé selon les revendications 7 et 8, caractérisé en ce que le solvant inerte est aromatique et/ou aliphatique et est de préférence du xylène.
